# EUROPEAN PATENT SPECIFICATION

(11) **EP 1 970 381 B1**
(45) Date of publication and mention of the grant of the patent: **18.11.2009**
(21) Application number: 07104132.1
(22) Date of filing: 14.03.2007
(51) Int. Cl.: C07K 14/47, C07K 14/59, C07K 14/705, C07K 14/81

(54) **Anti-cancer immune-modulating agent**
Immunmodulierendes Antikrebsmittel
Agent de modulation immunitaire anti-cancéreux

(43) Date of publication of application: 17.09.2008
(73) Proprietor: Montco Cancer Research B.V., 1962 XZ Heemskerk (NL)
(72) Inventor: Khachatrian, Robert, GLENDALE, CA 91204 (US); Kazanchian, Armen, GLENDALE, CA 91204 (US)
(74) Representative: van Heuvel, Margaretha

(56) References cited:
- WO-A-2006/107786
- FR-A1- 2 710 845
- CHEN XIN-YUAN ET AL: "Vaccination with viable human umbilical vein endothelial cells prevents metastatic tumors by attack on tumor vasculature with both cellular and humoral immunity." CLINICAL CANCER RESEARCH : AN OFFICIAL JOURNAL OF THE AMERICAN ASSOCIATION FOR CANCER RESEARCH 1 OCT 2006, vol. 12, no. 19, 1 October 2006 (2006-10-01), pages 5834-5840, XP002430888 ISSN: 1078-0432

## Description

The present invention relates to the field of treatment and/or prevention of cancer.

Currently, the problem of malignant diseases still is one of the most difficult ones humanity is faced to. Despite the technological achievements in early recognition, extensive preventive approaches along with life style modifications, new advances in chemotherapy and other therapeutic modalities, medicine still cannot offer a definite way to fight cancer.

It is well known that anti-cancer medications may substantially differ from each other and represent a variety of antibiotics, alkyl compositions, hormonal preparations, enzymes, different substances of animal and non-animal origin, etc. The mechanism of action of each group of anti-cancer preparations is different. The majority acts at the level of DNA/RNA by disrupting its normal replication and proliferation. Others interfere with cellular metabolism. There is no "universal" available agent acting at multiple levels.

Recent interest to therapeutic agents from embryonic source was justified by better efficacy, less toxicity and good tolerability. The downside of these agents (currently in use) is high antigen composition of the agent, which can trigger severe allergic reaction. Although some non-scientific issues arose in relation to these agents (ethical, religious etc.) nevertheless they form the most promising group of therapeutic agents in giving the final solution to treat and prevent malignancies.

The immunogenicity of tumor related antigens is weak compared to microbial, viral and other antigens. In order to express fully their immunogenicity, the antigens should be associated with major histocompatibility complex 1 and 2 antigens. In this way, T-cells are being triggered to fight the cancer cells recognized as foreign ones.

Most anticancer agents do not provide universal therapeutic and/or preventive measures, probably because they are designed to fight specific types of cancer.

The present invention aims at delivering universal protection against cancer development as well as to mobilize the body's resources to fight an existing malignant state. The anti-cancer composition disclosed in the present invention comprises particular embryonic antigens that also are found as cancer antigens in various tumor types. The composition surprisingly shows therapeutic activity against multiple cancer types. In addition, the composition of the invention can be used as a universal anti-cancer vaccine.

In a first aspect, the present invention provides a method for the preparation of a composition suitable for use as an anti-cancer agent (ACA). In the context of the invention, the term "anti-cancer" includes an cancer-preventive as well as a cancer-therapeutic activity of the composition. The composition is obtainable from a mixture of human umbilical cord tissue and animal embryonic tissues.

In particular, the method for the preparation of the composition comprises:
a) homogenizing animal embryonic tissue and human umbilical cord tissue at a temperature of ≤ -50°C, to obtain a powder;
b) diluting the powder with water, to obtain a homogenate;
c) irradiating the homogenate with UV light;
d) precipitating protein from the irradiated homogenate by adding ethanol to the homogenate and recovering the precipitate;
e) dissolving the precipitate in water to obtain a protein solution;
f) optionally removing debris from the solution;
g) subjecting the solution to ultrafiltration to obtain an ultrafiltrate;
h) drying the ultrafiltrate to obtain the anti-cancer composition.

Human umbilical cord tissue and any animal embryonic tissue may be used as starting material. In the context of the invention, the terms "animal embryonic tissue" and "animal embryos" refer to embryonic tissues as well as complete animal embryos, but exclude embryonic tissue or complete embryos from human origin.

Preferably, any animal species may be used as source of the embryonic tissue. Preferably, a mixture of embryos from different animal classes is used, for instance mammalian embryos, fish embryos and/or bird embryos. The use of embryos from different animal classes advantageously allows the provision of the appropriate levels of the different embryonic antigens in the composition according to the invention.

The tissues are preferably provided in a weight ratio of 1 part of animal embryonic tissue and 1-10 parts of human umbilical cord tissue, preferably 1 part of animal embryonic tissue and 2-5 parts of human umbilical cord tissue, more preferably 1 part of animal embryonic tissue and 3 parts of human umbilical cord tissue.

When animal embryos from different animal classes are used, they are preferably provided in the following weight ratios: 1 part of bird embryos, 1.5-2.5 parts of mammalian embryos, 1.5-2.5 parts of fish embryos, and mixed with 10-20 parts of human umbilical cord tissue. An especially preferred weight ratio is 1 part of bird embryos, 2 parts of mammalian embryos, 2 parts of fish embryos, and 15 parts of umbilical cord tissue.

In a preferred embodiment, the mammalian embryos are pig embryos, the bird embryos are chicken embryos and the fish embryos are trout embryos.

The embryos are preferably used when being at about a quarter of their gestational period. For instance, pig embryos may be at 26-30 days of gestation and chicken embryos may be at 6-8 days of gestation. Fish embryos may be at 4-6 days of gestation.

Human umbilical cord tissue is washed in running water directly after birth of the baby to remove blood residues. It may be stored frozen for a certain time period, e.g. 2-3 weeks, preferably up to 1 week, prior to further processing.

The animal embryos may also be stored frozen prior to use.

The (thawed) umbilical cord tissue is immersed in 70% ethanol for at least 1 hour. Also the animal embryos used are being immersed in 70% ethanol.

The umbilical cord tissue and embryos are cut into pieces of ≤ 1 cm and mixed in the desired ratio, e.g. as indicated above.

The tissue pieces are frozen at a temperature ≤ 50°, preferably in liquid nitrogen, and then are homogenized to achieve a powder condition. Any suitable homogenizer may be used, preferably a homogenizer allowing homogenization with high pressure. To control the quality of disintegration of the tissue, the presence of fragmented cellular nuclear as well as cytoplasmatic debris is judged by microscopic evaluation. The cells should be disintegrated as much as possible.

The thus-obtained powder is diluted about 1:2 to about 1:5 with water, to obtain a homogenate.

The water used in the present invention preferably is chemically pure water, e.g. distilled water.

The homogenate is irradiated with UV irradiation. This is preferably done in a shallow bed with a depth of 2 to 5 cm, more preferably with a depth 2 to 3 cm. The wavelength of the UV light preferably is 250 to 260 nm.

Any debris that is formed is removed from the UV-irradiated homogenate, for instance by centrifugation in a refrigerated centrifuge at 15,000 - 20,000g.

A protein fraction then is precipitated from the homogenate. This is preferably done by alcohol precipitation, more preferably using ethanol. To that end, the homogenate is mixed with 2 to 3 weight parts of 96% ethanol and protein is allowed to precipitate. Typically, complete precipitation is reached in about half an hour. The alcoholic mixture is centrifuged at high speed, e.g. at least at 20,000 g, to recover the precipitate.

The obtained precipitate is suspended in water and incubated under agitation to dissolve (most of) the precipitate. Preferably, the precipitate is suspended in 1 to 5 weight parts, more preferably in 3 to 5 weight parts, of water. A suitable time period for incubation for instance is 5 to 15 minutes. Any debris that may be present is removed, for instance by centrifugation at high speed (at least 20,000 g). The water preferably is distilled water or any other form of chemically pure water.

The resulting solution is subjected to ultrafiltration over a membrane with a molecular weight exclusion limit of 10-20 kDa, preferably of 10 kDa.

Optionally, prior to ultrafiltration the solution may be subjected to a prefiltration, for instance over a ceramic membrane with a pore size of 0.5-1.5 mm.

The resulting ultrafiltrate is dried, preferably by lyophilization.

The present invention also envisages separate processing of animal embryonic tissues and umbilical cord tissue and mixing in the appropriate ratios either at an intermediate stage of the process, for instance at the homogenate stage, or after dissolving the final dried product in water.

The composition obtainable by this method forms a second aspect of this invention.

The present invention provides a composition that advantageously contains a combination of embryonic antigens. In particular, the present invention discloses a composition comprising cancer embryonic antigen (CEA), alpha-fetoprotein (Alpha-FP), chorionic gonadotropin (CG), thyroxine-binding globulin (TBG), cancer antigen 125 (CA-125), and cancer antigen 19 (CA-19), respectively, in a ratio of 0.5-5 ng, 30-300 ng, 0.0005-0.015 IU, 15-150 ng, 5-45 U, and 30-300 U, respectively.

Preferably, the composition comprises cancer embryonic antigen (CEA), alpha-fetoprotein (Alpha-FP), chorionic gonadotropin (CG), thyroxine-binding globulin (TBG), cancer antigen 125 (CA-125), and cancer antigen 19 (CA-19), respectively, in a ratio of 1-2 ng, 90-110 ng, 0.001-0.01 IU, 40-60 ng, 10-20 U, and 70-110 U, respectively.

The above ratio is preferably measured on a solution of 5 mg of the dry composition diluted in 2 ml of distilled water.

The composition is suitable for use in therapy. In particular, such use relates to the prevention and/or treatment of all kinds of malignancies.

The composition of the invention comprises about 40-70% (w/w) protein, based on dry weight of the composition. The remainder is mainly carbohydrate. Most of the carbohydrate is conjugated to protein, and about 10-20% of the carbohydrate is free carbohydrate.

The dry composition is a sterile, white or light-yellow, cotton-like substance.

The composition of the invention may be administered subcutaneously, intravenously or topically.

For an injectable product, the composition of the invention is preferably mixed into normal saline (sterile 0.9% NaCl), to form an opaque, homogeneous fluid with a slightly yellow color, preferably having a concentration of 0.005-0.1% (w/w). Preferably, the injectable product is administered, optionally after dilution with normal saline, via subcutaneous injection in doses of 0.01-0.05 mg/kg. Preferred injection site is an inner surface of the forearm. The skin reaction at the site of injection reveals the ability of the body's immune system to respond to the composition.

Patients with established malignancies may be treated once every 3-6 months with the same dosing regime. It is recommended to give additional booster doses during the first month of the therapy in severe cases and/or to give extra doses in patients with advanced malignant states.

For prophylaxis, the composition of the invention typically is administered to an individual with risk factors and high predisposition to oncological diseases (family history, smoking, exposure to carcinogenic substances, history of previous cancer, high fibrinogenic states etc.) by one injection per year, also using doses of 0.01-0.05 mg/kg.

The composition of the invention also may be topically applied as a cream or an ointment. The concentration of ACA in the cream or ointment may be 0.1-0.5% It was surprisingly found that the composition has profound juvenile effect when applied to the skin.

The composition (ACA) is effectively used for the treatment of different kinds of human cancers including solid and non-solid tumors, leukemia, etc. and achieves steady therapeutic effect including full recovery from the cancerous state.

The composition (ACA) is also successfully used to prevent development of cancer in patients with heavy predisposition and risk factors to develop a cancerous state.

The composition of the invention is especially suitable to treat and/or to prevent neoplastic and pre-neoplastic lesions of the skin and mucous membranes.

The composition of the invention never triggered major side effects or complications upon administration.

### Example 1

### Preparation of ACA

Umbilical cord tissue after cleaning in running water (4 hours) was immersed in 70 % ethanol for 1.5 hours. Then it was mixed with pig embryos, trout embryos and chicken embryos, all immersed in alcohol, in following % of weight: 75 % human umbilical cord, 10% pig embryos, 10% fish embryos and 5% chicken embryos. The embryos of pig were at 26-30 days of gestation, embryos of trout were at 4-6 days of gestation and embryos of chicken were at 6-8 days of gestation. The tissues were cut into pieces ≤ 1 cm, mixed and frozen in liquid nitrogen.

The frozen pieces then were homogenized in a homogenizer with a high pressure force to achieve a powder condition.

The powder was then suspended in distilled water 1:4 and centrifuged with speed up to 400 rpm in a ball rotary centrifuge, where the contact surfaces were made from inert material. The quality of disintegration of the tissue was controlled by checking the presence of fragmented cellular nuclear as well as cytoplasmic debris, by microcopic evaluation.

The homogenate was subjected to UV irradiation in a shallow bed of about 3 cm for 30 minutes, after which it was again centrifuged in a refrigerated centrifuge with speed of 6000 rpm for a period of 15 minutes.

Proteins, among which glycoproteins, were precipitated from the supernatant by adding 2-3 weight parts 96 % ethanol and allowing the mixture to stand for at least half an hour. The mixture was then centrifuged at high speed up to 20.000 rpm for 20 minutes.

The precipitate was suspended 1:2 in distilled water and, after 10 minutes exposure in ball rotary centrifuge at 400 rpm and 37°C, was again centrifuged at a high speed (20.000 rpm) in zonal rotary centrifuge CR-65.

The supernatant was subjected to a macrofiltration step using a ceramic membrane with a pore size of 1.4 mm (Jiangsu Jiuwu Hi-tech Co. Ltd, China)). Then, the filtrate was subjected to ultrafiltration using a modified polyethersulfone membrane with a molecular weight exclusion limit of 10 kDa (AC080R01, AMC, USA). The resulting ultrafiltrate was subjected to drying by lyophilization.

Freezing was done in a rotatory sterile chamber at -25 to -30 °C for 2 hours. The preparation then was exposed to -38 to -40 °C in a freezing chamber for 24 hours.

The final product was obtained by gradual increase of the temperature from -40 to room temperature over 28-30 hours.

### Example 2

### Composition and characteristics of ACA

In the lyophilized product, the concentration of protein constitute about 60% of the weight of ACA (by spectrophotometer analysis). About 1.5% constitutes free carbohydrates, where 8-10% is conjugated carbohydrates.

There are some enzymes and minerals present in the product, measured when dissolving 5 mg dry product in 2 ml distilled water:

| | |
|---|---|
| Alanine amino transferase (ALT) | 1.4 U/l |
| Aspartate amino transferase (AST) | 71.9 mmol/l |
| Lactate dehydrogenase (LDH) | 3.0 U/l |
| Alkaline phosphatase (APT) | 24.7 U/l |
| Mg | 1.7 mmol/l, |
| K | 0.96 mmol/l, |
| Ca | 0.65 mmol/l, |
| P | 0.52 mmol/l. |

The monoclonal immunoassay revealed presence of the following cancer embryonic compounds (the sample size was 5 mg of the composition dissolved in 2 ml of distilled water):

| | |
|---|---|
| Cancer embryonic antigen (CEA) | 1.4-1.7 ng/ml |
| Alpha-fetoprotein (Alpha-FP) | 95-99 ng/ml |
| Chorionic gonadotropin (CG) | 0.0022-0.0055 IU/ml |
| Thyroxine-binding globulin (TBG) | 45-50 ng/ml |
| Cancer antigen 125 (CA-125) | 13.5-14.3 U/ml |
| Cancer antigen 19 (CA-19) | 79.8-102.2 U/ml |

CG, TBG and AFP were detected by CMIA (chemiluminescent immuno-assay on Abbot Architect analyzer) and CEA, CA-19 and CA-125 were detected by MEIA (microparticle enzyme immuno-assay on Abbot AxSYM analyzer).

These embryonic antigens are similar to multiple cancer antigens and cross-react with them.

Along with embryonic antigens there are some other types of proteins in ACA which were detected by electrophoresis, chromatography, etc. These proteins most probably were responsible for some interferon-like, anti-virus and anti-mutagen characteristics of the product (ACA).

ACA is a porous, soft substance having a light yellow and/or white color without any smell or taste.

The injectable product is a 0.02%, opaque, homogeneous fluid with a slightly yellow color.

### Example 3

### Toxicity, mutagenesis

The toxicity experiments were performed on 24 dogs who were receiving doses of ACA of one human therapeutic dose, and of 10-fold and 25-fold of a human therapeutic dose.

The dogs were monitored 7, 15 and 30 days after injections. The baseline parameters were registered before the start of the experiment (EKG, temperature, RR, urine analyses, CBC, full chem.-21 including AST, ALT, APT, LDH etc.

Mutagenesis was tested on rats according to WHO recommendations. The ACA was introduced to the rats 5-15 days before application of benzpirena and or 7.12 dimethilbenzantracena. The study of rats bone marrow showed definite decrease of megakariocytes with chromosomal aberrations (p<0.001) in comparison with control group (only cancerogens).

### Example 4

### Treatment of patients with ACA

Preventive approach: to the high risk group of the patients - one vial s/q once a year.

Treatment with ACA: one vial per each injection, four times per year. The second injection should be given a month after the first one, then every 6 months.

The site of first injection usually develops local reaction in the way of redness 3-6 cm with some elevation and induration, which disappears after 48 hours. The local reaction can give as some idea about anti-cancer immune status of the patient, based on the fact that it is basically a set of cancer antigens. So if there is an intense local reaction, definitely the ability of the immune system to distinguish and react to cancer is in good shape.

The data obtained with therapy and prophylaxis are shown in Tables 1 and 2, respectively.

**Table 1**

| ***Comparative data on effect of ACA for treatment of different cancer states*** | | | | |
|---|---|---|---|---|
| ACA treatment group (5 years follow-up) | | | Conventional treatment group | |
| Disease | Pts | % of patients improved | Pts | % of patients improved |
| Breast CA (no surgery) | 120 | 76% | 115 | 32% |
| Stomach CA | 180 | I-II stage 88% III-IV stage - 76% | 160 | 42% |
| Leukemia (CLL) | 118 | 91% | 88 | 56% |
| Lung CA | 192 | 71% | 145 | 45% |
| Prostate CA | 128 | 82% | 114 | 52% |
| Colon CA | 136 | I-II stage -91% III-IV stage - 78% | 119 | 56% |
| Total | 874 | | 741 | |

**Table 2**

| ***Comparative data on ACA as a vaccine in prevention of cancer states in first line relatives of patients with diagnosed malignancies*** The participants were followed for 7 years, or until being diagnosed with cancer. | | | | |
|---|---|---|---|---|
| Type of CA in first line relatives | Participants 1 s/q injection per year | Incidents of CA in prevention group | Participants without vaccination | Incidents of CA in non-prevention group |
| Stomach, Pancreatic CA, Colon CA | 1132 | 2-5% | 907 | 17-23 % |
| Leukemia/CLL | 366 | 6% | 298 | 19% |
| Breast CA | 476 | 5% | 421 | 21% |
| Prostate CA | 397 | 3% | 323 | 15% |
| Total | 2371 | | 1949 | |

## Claims

1. A method for the preparation of a composition suitable as an anti-cancer agent comprising:
a) homogenizing animal embryonic tissue excluding embryonic tissue or complete embryos from human origin and human umbilical cord tissue at a temperature of ≤ -50°C, to obtain a powder;
b) diluting the powder with water, to obtain a homogenate;
c) irradiating the homogenate with UV light;
d) precipitating protein from the irradiated homogenate by adding ethanol to the homogenate and recovering the precipitate;
e) dissolving the precipitate in water to obtain a protein solution;
f) optionally removing debris from the solution;
g) subjecting the solution to ultrafiltration to obtain an ultrafiltrate;
h) drying the ultrafiltrate to obtain the anti-cancer composition.

2. The method according to claim 1, wherein the tissues are provided in a weight ratio of 1 part of animal embryonic tissue and 1-10 parts of human umbilical cord tissue, preferably in a weight ratio 1 part of animal embryonic tissue and 2-5 parts of human umbilical cord tissue, more preferably in a weight ratio 1 part of animal embryonic tissue and 3 parts of human umbilical cord tissue.

3. The method according to claim 1 or 2, wherein the animal embryonic tissue comprises non-human mammalian, fish and/or bird embryos.

4. The method according to claim 3, wherein the animal embryonic tissue comprises pig, trout and/or chicken embryos.

5. The method according to claim 3 or 4, wherein the tissues are provided in a weight ratio of 1 part of bird embryos, 1.5-2.5 parts of mammalian embryos, 1.5-2.5 part of fish embryos and 10-20 parts of human umbilical cord tissue, preferably in a weight ratio of 1 part of bird embryos, 2 parts of mammalian embryos, 2 parts of fish embryos, and 15 parts of umbilical cord tissue.

6. The method according to any one of claims 3 to 5, wherein the mammalian, bird and fish embryos are at about a quarter of their gestational period.

7. The method according to claim 4 or 5, wherein the pig embryos are at 26-30 days of gestation, the fish embryos are at 4-6 days of gestation and the chicken embryos are at 6-8 days of gestation.

8. The method according to any one of the preceding claims, wherein the precipitation of protein comprises mixing 1 weight part of the homogenate with 2 to 3 weight parts of 96% ethanol.

9. The method according to any one of the preceding claims, wherein ultrafiltration occurs over a membrane with a molecular weight exclusion limit of 10-20 kDa.

10. A composition comprising cancer embryonic antigen (CEA), alpha-fetoprotein (Alpha-FP), chorionic gonadotropin (CG), thyroxine-binding globulin (TBG), cancer antigen 125 (CA-125), and cancer antigen 19 (CA-19), respectively, in a ratio of 0.5-5 ng, 30-300 ng, 0.0005-0.015 IU, 15-150 ng, 5-45 U, and 30-300 U, respectively.

11. An aqueous composition comprising cancer embryonic antigen (CEA), alpha-fetoprotein (Alpha-FP), chorionic gonadotropin (CG), thyroxine-binding globulin (TBG), cancer antigen 125 (CA-125), and cancer antigen 19 (CA-19), respectively, in a ratio of 0.5-5 ng, 30-300 ng, 0.0005-0.015 IU, 15-150 ng, 5-45 U, and 30-300 U, respectively, and water up to 1 ml.

12. The composition of claim 10 or 11 for use in therapy.

13. The composition of claim 10 or 11 for use in therapy or prophylaxis of cancer.

## Patentansprüche

1. Ein Verfahren für die Herstellung einer Zusammensetzung, die als ein Anti-Krebs-Mittel verwendbar ist, das umfasst:
a) Homogenisieren von embryonalem Tiergewebe, das embryonales Gewebe oder komplette Embryos menschlichen Ursprungs ausschließt, und menschlichem Nabelschnurgewebe bei einer Temperatur von ≤ -50°C, um ein Pulver zu erhalten;
b) Verdünnen des Pulvers mit Wasser, um ein Homogenat zu erhalten;
c) Bestrahlen des Homogenats mit UV-Licht;
d) Ausfällen von Protein aus dem bestrahlten Homogenat durch Hinzufügen von Ethanol zu dem Homogenat und Zurückgewinnen der Ausfällung;
e) Auflösen der Ausfällung in Wasser, um eine Proteinlösung zu erhalten;
f) optionales Entfernen von Debris aus der Lösung;
g) Unterziehen der Lösung einer Ultrafiltration, um ein Ultrafiltrat zu erhalten;
h) Trocknen des Ultrafiltrats, um eine Anti-Krebs Zusammensetzung zu erhalten.

2. Das Verfahren gemäß Anspruch 1, wobei die Gewebe in einem Gewichtsverhältnis aus 1 Teil des embryonalen Tiergewebes und 1-10 Teilen des menschlichen Nabelschnurgewebes, bevorzugt aus einem Gewichtsverhältnis von 1 Teil des embryonalen Tiergewebes und 2-5 Teilen des menschlichen Nabelschnurgewebes, bevorzugter aus einem Gewichtsverhältnis von 1 Teil embryonalen Tiergewebes und 3 Teilen menschlichem Nabelschnurgewebe bereitgestellt werden.

3. Das Verfahren gemäß Anspruch 1 oder 2, wobei das embryonale Tiergewebe nicht-menschliche Säugetier-, Fisch- und/oder Vogelembryos umfasst.

4. Das Verfahren gemäß Anspruch 3, wobei das embryonale Tiergewebe Schweine-, Forellen- und/oder Hühnerembryos umfasst.

5. Das Verfahren gemäß Anspruch 3 oder 4, wobei die Gewebe in einem Gewichtsverhältnis aus 1 Teil Vogelembryos, 1.5-2.5 Teilen Säugetierembryos, 1.5-2.5 Teile Fischembryos und 10-20 Teilen menschlichem Nabelschnurgewebe, bevorzugt in einem Gewichtsverhältnis aus 1 Teil Vogelembryos, 2 Teilen Säugetierembryos, 2 Teilen Fischembryos und 15 Teilen menschlichem Nabelschnurgewebe bereitgestellt werden.

6. Das Verfahren gemäß einem der Ansprüche 3 bis 5, wobei die Säugetier-, Vogel- und Fischembryos bei ungefähr einem Viertel ihrer Gestationszeit sind.

7. Das Verfahren gemäß Anspruch 4 oder 5, wobei die Schweineembryos bei 26-30 Tagen der Gestation, die Fischembryos bei 4-6 Tagen der Gestation und die Hühnerembryos bei 6-8 Tagen der Gestation sind.

8. Das Verfahren gemäß einem der vorangegangenen Ansprüche, wobei die Ausfällung des Proteins das Mischen von 1 Gewichtsteil des Homogenats mit 2 bis 3 Gewichtsteilen 96igem Ethanol umfasst.

9. Das Verfahren gemäß einem der vorangegangenen Ansprüche, wobei die Ultrafiltration über eine Membran mit einer Ausschlussgrenze des Molekulargewichts von 10-20 kDa erfolgt.

10. Eine Zusammensetzung, die carcinoembryonales Antigen (CEA), Alpha-Fetoprotein (Alpha-FP), Choriongonadotropin (CG), Thyroxin-bindendes Globulin (TBG), Cancer-Antigen 125 (CA-125) bzw. Cancer-Antigen 19 (CA-19) in einem Verhältnis von 0.5-5 ng, 30-300 ng, 0.0005-0.015 IU, 15-150 ng, 5-45 U bzw. 30-300 U umfasst.

11. Eine wässrige Zusammensetzung die carcinoembryonales Antigen (CEA), Alpha-Fetoprotein (Alpha-FP), Choriongonadotropin (CG), Thyroxin-bindendes Globulin (TBG), Cancer-Antigen 125 (CA-125) bzw. Cancer-Antigen 19 (CA-19) in einem Verhältnis von 0.5-5 ng, 30-300 ng, 0.0005-0.015 IU, 15-150 ng, 5-45 U bzw. 30-300 U und bis zu 1 ml Wasser umfasst.

12. Die Zusammensetzung nach Anspruch 10 oder 11 für die Verwendung in der Therapie.

13. Die Zusammensetzung nach Anspruch 10 oder 11 für die Verwendung in der Therapie oder Prophylaxe von Krebs.

## Revendications

1. Procédé de préparation d'une composition adaptée en tant qu'agent anticancéreux, qui comprend :
a) l'homogénéisation de tissu embryonnaire animal, à l'exclusion de tissu embryonnaire ou d'embryons complets d'origine humaine, et de tissu de cordon ombilical humain à une température ≤ -50 °C, pour obtenir une poudre ;
b) la dilution de la poudre avec de l'eau, pour obtenir un homogénat ;
c) l'irradiation de l'homogénat avec de la lumière UV ;
d) la précipitation de la protéine de l'homogénat irradié par adjonction d'éthanol à l'homogénat et la récupération du précipité ;
e) la dissolution du précipité dans l'eau pour obtenir une solution protéique ;
f) l'élimination éventuelle des débris de la solution ;
g) la soumission de la solution à une ultrafiltration pour obtenir un ultrafiltrat ;
h) le séchage de l'ultrafiltrat pour obtenir la composition anticancéreuse.

2. Procédé selon la revendication 1, dans lequel les tissus embryonnaires sont fournis en un rapport en poids de 1 partie de tissu embryonnaire animal et 1 à 10 parties de tissu de cordon ombilical humain, de préférence en un rapport en poids de 1 partie de tissu embryonnaire animal et 2 à 5 parties de tissu de cordon ombilical humain, de préférence encore en un rapport en poids de 1 partie de tissu embryonnaire animal et 3 parties de tissu de cordon ombilical humain.

3. Procédé selon la revendication 1 ou 2, dans lequel le tissu embryonnaire animal comprend des embryons de mammifères non humains, de poissons et/ou d'oiseaux.

4. Procédé selon la revendication 3, dans lequel le tissu embryonnaire animal comprend des embryons de porc, de truite et/ou de poulet.

5. Procédé selon la revendication 3 ou 4, dans lequel les tissus sont fournis en un rapport en poids de 1 partie d'embryons d'oiseaux, 1,5 à 2,5 parties d'embryons de mammifères, 1,5 à 2,5 parties d'embryons de poisson et 10 à 20 parties de tissu de cordon ombilical humain, de préférence en un rapport en poids de 1 partie d'embryons d'oiseaux, 2 parties d'embryons de mammifères, 2 parties d'embryons de poissons, et 15 parties de tissu de cordon ombilical.

6. Procédé selon l'une quelconque des revendications 3 à 5, dans lequel les embryons de mammifères, d'oiseaux et de poissons se trouvent environ au quart de leur période gestationnelle.

7. Procédé selon la revendication 4 ou 5, dans lequel les embryons de porc se trouvent à 26 à 30 jours de gestation, les embryons de poisson se trouvent à 4 à 6 jours de gestation, et les embryons de poulet se trouvent à 6 à 8 jours de gestation.

8. Procédé selon l'une quelconque des revendications précédentes, dans lequel la précipitation de la protéine comprend le mélange de 1 partie en poids de l'homogénat avec 2 à 3 parties en poids d'éthanol à 96 %.

9. Procédé selon l'une quelconque des revendications précédentes, dans lequel l'ultrafiltration a lieu sur une membrane avec une limite d'exclusion de masse moléculaire de 10 à 20 kDa.

10. Composition comprenant l'antigène carcino-embryonnaire (CEA), l'alpha-fétoprotéine (Alpha-FP), la gonadotrophine chorionique (CG), une globuline liant la thyroxine (TBG), l'antigène de cancer 125 (CA-125), et l'antigène de cancer 19 (CA-19), respectivement, en un rapport de 0,5 à 5 ng, 30 à 300 ng, 0,0005 à 0,015 UI, 15 à 150 ng, 5 à 45 U, et 30 à 300 U, respectivement.

11. Composition aqueuse comprenant un antigène carcino-embryonnaire (CEA), l'alpha-fétoprotéine (Alpha-FP), la gonadotrophine chorionique (CG), une globuline liant la thyroxine (TBG), l'antigène de cancer 125 (CA-125), et l'antigène de cancer 19 (CA-19), respectivement, en un rapport de 0,5 à 5 ng, 30 à 300 ng, 0,0005 à 0,015 UI, 15 à 150 ng, 5 à 45 U, et 30 à 300 U, respectivement, et de l'eau jusqu'à 1 mL.

12. Composition selon la revendication 10 ou 11, destinée à être utilisée en thérapie.

13. Composition selon la revendication 10 ou 11, destinée à être utilisée pour la thérapie ou la prophylaxie d'un cancer.
